(19) Europäisches Patentamt
European Patent Office
Office européen des brevets

(11) Publication number: 0 012 543
B1

(12) EUROPEAN PATENT SPECIFICATION

(45) Date of publication of patent specification: 11.05.83

(21) Application number: 79302711.1

(22) Date of filing: 28.11.79

(51) Int. Cl.³: C 07 D 317/12, A 61 K 7/00, C 11 D 7/26

# (54) Compositions containing 1,3-dioxolanes as emollients.

(30) Priority: 30.11.78 GB 4663678

(43) Date of publication of application: 25.06.80 Bulletin 80/13

(45) Publication of the grant of the patent: 11.05.83 Bulletin 83/19

(84) Designated Contracting States: AT BE CH DE FR GB IT LU NL SE

(56) References cited:
DE - A - 2 526 675
DE - A - 2 707 875
DE - B - 1 287 060
GB - A - 1 235 129
GB - A - 1 549 213
US - A - 3 855 290
US - A - 4 031 112
US - A - 4 124 641

(73) Proprietor: IMPERIAL CHEMICAL INDUSTRIES PLC
Imperial Chemical House Millbank
London SW1P 3JF (GB)

(72) Inventor: Greenshields, James Nairn
27 Fernwood
Marple Bridge Stockport, Cheshire (GB)
Inventor: Sherman, Albert Herman
1426 Fresno Road
Wilmington Delaware 19803 (US)

(74) Representative: Cooke, Edward Graham et al,
Imperial Chemical Industries Limited Legal Dept. Patents Thames House North Millbank
London SW1P 4QG (GB)

(56) References cited:
ULLMANNS ENCYCLOPÄDIE DER TECHNISCHEN CHEMIE, 3rd edition, Vol. 18, 1967, URBAN AND SCHWARZENBERG, München, Berlin, Wien, page 562

The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 012 543 B1

Courier Press, Leamington Spa, England

Compositions containing 1,3-dioxolanes as emollients.

This invention relates to compositions containing certain 1,3-dioxolanes which are useful in cosmetic, toiletry or domestic cleaning applications and in which the presence of the 1,3-dioxolane confers emolliency properties.

In German Patent publication 2 707 875 bicyclic acetals are described, based on pentaerythritol and $C_8$—16 aldehydes. The products are foam inhibitors in the presence of surfactants.

From UK Patent 1 253 129, ketals are known which include a 1,3-dioxolane ring, in which the 4 and 5 positions are occupied by the group $—CH_2—CH_2—$.

US Patent 4 124 641 describes surface active acetals, which may be used in simple aqueous cleaning solutions Cyclic acetals however are not considered.

Cyclic acetals and ketals of polyalcohols are discussed in US Patent 4 031 112. These products have spare hydroxyl groups available for oxyalkylation and the oxyalkylated product is an emulsifier.

Finally, UK Patent 1 549 213 (equivalents are Netherlands Application 7605 563 and German Application 2 526 675) describes cyclic acetals and ketals as inflammation inhibitors for cosmetic preparations. The number of carbon atoms attached to the 2-position of the dioxolane ring may be as high as twelve. The emollient properties of the dioxolanes is not identified and indeed, added emollients may be necessary (e.g. Example 3 therein).

It has now been found that certain dioxolanes, several of which may be known from the above publications, have the hitherto unexpected property of emolliency. The term emolliency means a particular skin feel when rubbed on the skin which is desirable in the formulation of cosmetics and other compositions which come into contact with the skin.

Accordingly, the present invention provides a cosmetic, toiletry or domestic cleaning composition which is an emulsion or a stick composition containing active ingredients, in which there is additionally present, as an emollient, an effective amount of

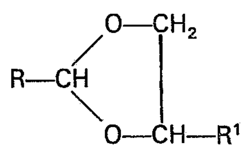

wherein

R is an aliphatic chain having from 7 to 30 carbon atoms; and

$R^1$ is methyl or hydrogen.

Incorporation of these dioxolanes into compositions of the type referred to above adds emollient properties to such compositions.

The aliphatic chain R preferably contains from 8 to 20 carbon atoms and examples include alkyl groups containing 10, 11, 12, 13, 14, 15 and 16 carbon atoms which groups may be straight chain or branched. An especially preferred form of R is a mixture of $C_{12}$ and $C_{14}$ alkyl groups. It is further preferred that each of these $C_{12}$ and $C_{14}$ alkyl groups comprises a mixture of straight and branched chains, particularly $\alpha$-branched chains.

Suitable aldehydes for use in preparing acetals according to the present invention are conveniently prepared from olefines, especially $\alpha$-olefines, by the oxo-process, i.e. hydrocarbonylation of the double bonded carbon atoms. An especially preferred oxo-aldehyde, prepared from mixed $C_{12}$ and $C_{14}$ $\alpha$-olefines, comprises a 70:30 mixture of $C_{12}$ and $C_{14}$ alkyl chains approximately 50% of which are straight and 50% are branched.

The 1,3-dioxolane is made by reacting the appropriate aldehyde or mixed aldehydes with the appropriate diol, preferably in the presence of an acid catalyst, or may alternatively be made by what may be described as an acetal-interchange reaction using the diol and for example the di (lower alkyl), for example dimethyl, acetal of the aldehyde.

When an aldehyde is reacted with the appropriate diol, the water formed can be removed by distillation, vacuum stripping or by the use of an entraining solvent or any known method.

The compositions according to the present invention may take a variety of forms, based either on emulsion and/or stick compositions capable of softening and spreading an application of heat or pressure. An important group comprises cosmetic formulations used for application to the skin. Thus they may be incorporated in creams and lotions for hair and skin care, astringents, cleansers, shaving aids, after-shaves, sun tan preparations, antiperspirants, deodorants, manicure, pedicure and lotion specialities, pigmented make-up, lipsticks, shampoo and bath specialities and light duty household cleansing agent which come into contact with the skin. They may be used in admixture with other materials having emollient properties.

The invention is illustrated by the following Examples in which all parts are by weight except where otherwise stated. Products marked with an asterisk are given their trade names.

Preparation of acetals is first described, not only of those for use according to the invention, but

also for use in comparative tests.

Preparation of Acetals

*Preparation* 1

212 Parts of a mixed $C_{13}$ and $C_{15}$ 'oxo' aldehyde, containing a 70:30 mixture of $C_{12}$ and $C_{14}$ alkyl chains in which 47% are linear and 53% are branched (80% of the branched chains having a $\alpha$-methyl group), 84 parts of 1,2-propylene glycol, 0.4 part of p-toluenesulphonic acid monohydrate and 100 ml. of toluene are charged to a 1-litre three-necked round bottom flank equipped with stirrer, nitrogen inlet, thermometer and Dean-Stark trap topped with a water condenser. Stirring and nitrogen flow are started and the mixture is heated to reflux (ca. 30 min.) and maintained at reflux until the theoretical amount of water (18.0 parts) has separated (ca. 2 hours). The mixture is cooled to room temperature and then washed sequentially with 200 parts of 5% sodium bicarbonate solution and 200 parts of water. The toluene solution is stirred with "Darco* KB" activated carbon (2.5 parts) for about 20 minutes, filtered over Super Cel* filtering aid to remove the activated charcoal and the filtrate evaporated in vacuo (3 hours at 15 mm Hg) at a pot temperature of about 70°C to give 235.0 parts (91%) of the desired 1,2-propylene glycol acetal as a pale yellow oil. Acid number: 1.7; GLC analysis: no 1,2-propylene glycol present; NMR: no aldehyde CH resonance.

*Preparation 2*

212 Parts of the mixed $C_{13}$ and $C_{15}$ aldehyde used in Preparation 1, 84 parts of 1,2-propylene glycol and 0.4 part of p-toluenesulphonic acid monohydrate are charged to a 1-litre three-necked round bottom flask equipped with stirrer, nitrogen sparge inlet submerged 12 mm below the liquid surface, thermometer, and a Dean-Stark trap topped with a water condenser. Stirring and nitrogen flow (2.2 litres/min.) are started and mixture is heated to 100°C during 20 minutes and maintained at 100°C until 18.0 parts of condensate collect in the trap (2.0 hours). The mixture is cooled to room temperature and then washed sequentially with 200 parts of 5% sodium bicarbonate solution and 200 parts of water. The acetal is then stripped at 80°C/1 mm Hg for 0.75 hour. Darco* KB charcoal (2.5 parts, 1% by wt.) is added to the acetal, the mixture stirred at ambient temperature for 20 minutes and then filtered over Supercel* to remove the Darco*. The yield of 1,2-propylene glycol acetal, obtained as a pale yellow oil, is 253.3 parts (94%). Acid number: 3.99, GLC analysis: no. 1,2-propylene glycol present; NMR: no aldehyde CH resonance.

*Preparation 3*

Using the procedure described in Preparation 1, 198 parts of a mixed $C_{13}$ and $C_{15}$ aldehyde containing linear and x-methyl chains is treated with 93 parts of ethylene glycol and 0.3 parts of p-toluenesulphonic acid in refluxing toluene to azeotrope water. After washing with aqueous bicarbonate and water, and evaporation of solvent, the acetal product is a pale yellow liquid with low odour. It shows no aldehyde by NMR.

*Preparation 4*

Using the procedure of Preparation 1, 84.8 parts of the mixed $C_{13}$ and $C_{15}$ aldehyde used in Preparation 1 are treated with 14.6 parts of neopentyl glycol and 0.2 part of p-toluenesulphonic acid in refluxing toluene to azeotrope water. After washing with water and evaporating the solvent, the acetal product is a pale yellow liquid containing no free aldehyde.

*Preparation 5*

A cyclic acetal is prepared by condensation of 25.6 parts of octanol (derived by hydroformylation of polymer gasoline heptene), 16.7 parts of 1,2-propylene glycol and 0.5 part of 80% phosphoric acid at 40—50°C and 15 mm Hg for approximately 20 hours. The product is a water white liquid with a pleasant skin feel.

*Preparation 6*

Using the reaction conditions of Preparation 5, 24 parts of a mixed $C_{15}$ and $C_{17}$, linear and x-methyl, 'oxo' aldehyde, 8.35 parts of 1,2-propylene glycol and 0.6 part of 80% phosphoric acid are condensed to give a cyclic acetal. The product is a water white clear liquid with a very pleasant skin feel.

*Preparation 7*

Using the procedure of Preparation 1, 396 parts of the mixed $C_{13}$ and $C_{15}$ aldehyde used in Preparation 1 is reacted with 276 parts of glycerine and 1.3 parts of p-toluenesulphonic acid and in refluxing toluene to azeotrope water. After washing with water and evaporating solvent, the product is an odourless liquid which shows no free aldehyde by NMR.

Other polyols, 1,3-propanediol and 1,4-butanediol were converted to acetals of the aldedhyde of preparation by similar methods.

*Evaluation of Emollients*

The following basic composition is prepared:

| | Oil Phase |
|---|---|
| 7.0 parts | Acetal from Preparation above |
| 4.0 parts | Glyceryl Monostearate (Atmul* 84) |
| 3.0 parts | Stearic Acid |

| | Water Phase |
|---|---|
| 4.0 parts | Propylene Glycol |
| 0.5 part | Triethanolamine |
| 0.4 part | Sodium Lauryl Sulphate |
| 81.1 parts | Water |

The emollient to be tested is stirred with the other ingredients of the oil phase while heating to 70°C. Meanwhile, ingredients of the water phase are stirred and heated to 72°C. The water phase is then added gradually to the stirred oil phase, and the total mixture is stirred while it cools to room temperature. The emulsion (lotion or cream) is allowed to age for 48 hours at room temperature before evaluation.

For evaluation the subject applies a small amount of the lotion to the inner arm, rubs it into the skin and rates five characteristics: ease of application, moistness, smoothness, greasiness and tackiness. Numerical values are given as follows: poor = 0, average or "not sure" = 1, and good = 2. This is done by an experienced panel of 10 persons and ratings for each emollient are the mean for the 10 subjects. With this system, the highest possible rating is 10; the average emollient has a rating of 5; and the worst score is zero. Emollients of the present invention have ratings above 8.

Specific examples of acetals of fatty aldehydes are listed following under the name of the polyol and preparation number. The aldehyde in each case was the mixed $C_{13}$ and $C_{15}$ aldehyde used in Preparation 1. Structures and emollient ratings (0—10 scale) are as shown in the following Table:

| Hydroxyl Compound | Preparation | Emollient Rating |
|---|---|---|
| Ethylene glycol | 3 | 8.6 |
| 1,2-Propylene glycol | 1 | 8.1 |
| Other Hydroxyl compounds for comparision | | |
| 1,3-Propanediol | — | 6.3 |
| Neopentyl glycol | 4 | 7.7 |
| 1,4-Butanediol | — | 5.8 |
| Pentaerythritol | — | 6.3 |
| Glycerol | 7 | 5.8 |

*Preparation of a Deodorant Stick*

The following ingredients are mixed, melted and stirred until homogeneous:

| Amount (parts) | Ingredient |
|---|---|
| 18 | $C_{17}H_{35}COOC_3H_6O(C_2H_4O)_{25}H$ |
| 2 | $C_{17}H_{35}O(C_2H_4O)_{100}H$ |
| 10 | Volatile Silicone |
| 20 | Rehydrol* (aluminium chlorhydrate + propylene glycol) |
| 30 | Stearyl Alcohol |
| 20 | $C_{13}/_{15}$ aldehyde-ethylene glycol acetal (2-alkyl-1,3-dioxolane) |

This mixture is cooled to 55—60°C, poured into a mould and cooled to solidify. The deodorant stick is non-greasy, shows no whitening, and has good payoff (deposition on skin). These are all desirable qualities.

**Claims**

1. A cosmetic, toiletry or domestic cleaning composition which is an emulsion or a stick composition containing active ingredients, in which there is additionally present, as an emollient, an effective amount of a 1,3-dioxolane having the formula

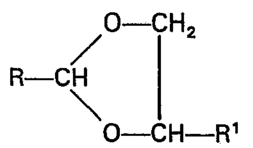

wherein

R is an aliphatic chain having from 7 to 30 carbon atoms; and

$R^1$ is methyl or hydrogen

2. A composition according to Claim 1 in which in the formula R is an aliphatic chain comprising a mixture of $C_{12}$ and $C_{14}$ alkyl groups.

3. A composition according to Claim 2 in which in the formula of Claim 1 R is a 70:30 mixture of $C_{12}$ and $C_{14}$ alkyl chains, 50 $\pm$ 5% of which are straight chain and 50 $\mp$ 5% are branched.

**Patentansprüche**

1. Zusammensetzung für kosmetische oder Toilettenzwecke oder für die Haushaltsreinigung in Form einer Emulsion oder einer stangen- bzw. stiftförmigen Masse, die wirksame Bestandteile enthält, worin außerdem als Erweichungsmittel ine wirksame Menge eines 1,3-Dioxolans der Formel:

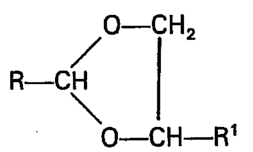

worin

R eine aliphatische Kette mit 7 bis 30 Kohlenstoffatomen ist und

$R^1$ eine Methylgruppe oder eine Wasserstoffatom ist, enthalten ist.

2. Zusammensetzung nach Anspruch 1, wobei in der Formel R eine aliphatische Kette ist, die aus einer Mischung von $C_{12}$- und $C_{14}$-Alkylgruppen besteht.

3. Zusammensetzung nach Anspruch 2, wobei in der Formel von Anspruch 1 R eine Mischung von $C_{12}$- und $C_{14}$-Alkylketten im Verhältnis 70:30 ist und wobei von den Alkylketten jeweils (50 $\pm$ 5)% geradkettig und (50 $\mp$ 5)% verzweigt sind.

**Revendications**

1. Composition cosmétique, de toilette ou de nettoyage domestique qui est une émulsion ou une composition en bâton contenant des constituants actifs, dans laquelle est présente en outre, comme émollient, une quantité efficace d'un 1,3-dioxolane de formule:

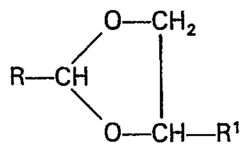

où

R est une chaîne aliphatique comptant 7 à 30 atomes de carbone et

$R^1$ est méthyle ou hydrogène.

2. Composition suivant la revendication 1, dans laquelle dans la formule R est une chaîne aliphatique comprenant un mélange de radicaux alcoyle en $C_{12}$ et $C_{14}$.

3. Composition suivant la revendication 2, dans laquelle dans la formule de la revendication 1, R est un mélange 70:30 chaînes alcoyle en $C_{12}$ et $C_{14}$, dont 50 $\pm$ 5% sont des chaînes droites et 50 $\mp$ 5% sont ramifiées.